# EUROPÄISCHE PATENTSCHRIFT

(11) **EP 1 096 960 B1**
(45) Veröffentlichungstag und Bekanntmachung des Hinweises auf die Patenterteilung: **26.03.2003**
(21) Anmeldenummer: 99947228.5
(22) Anmeldetag: 20.07.1999
(51) Int. Cl.: A61L 11/00, A61L 2/06

(54) **VORRICHTUNG ZUR STERILISATION VON KONTAMINIERTEN MATERIALIEN**
DEVICE FOR STERILIZING CONTAMINATED MATERIALS
DISPOSITIF DE STERILISATION DE MATIERES CONTAMINEES

(30) Priorität: 23.07.1998 DE 19833023
(43) Veröffentlichungstag der Anmeldung: 09.05.2001
(73) Patentinhaber: Göldner, Helmut, 31633 Leese (DE)
(72) Erfinder: Göldner, Helmut, 31633 Leese (DE)
(74) Vertreter: Braun, Dieter, Dipl.-Ing.
(86) Internationale Anmeldenummer: DE9902211
(87) Internationale Veröffentlichungsnummer: WO00004934

(56) Entgegenhaltungen:
- EP-A- 0 672 426
- WO-A-98/48853
- DE-A- 2 952 544
- DE-C- 3 938 546
- US-A- 3 464 342
- DATABASE WPI Section Ch, Week 199932 Derwent Publications Ltd., London, GB; Class D14, AN 1999-374391 XP002123068 & JP 11 137644 A (THERMAL KK), 25. Mai 1999 (1999-05-25)

## Beschreibung

Die Erfindung bezieht sich auf eine Vorrichtung zur Desinfektion bzw. Sterilisation von kontaminierten, insbesondere infizierten Materialien, mit einer ersten Förderschnecke, die innerhalb einer Vorbehandlungskammer verläuft und einer zweiten Förderschnecke, die am Ende der ersten Förderschnecke angeordnet ist und innerhalb einer Behandlungskammer verläuft, wobei Mittel für eine Abdichtung der beiden Endbereiche der Behandlungskammer zum Aufbau eines Überdruckes vorhanden sind, in die Behandlungskammer Energie definiert einleitbar ist, Wasserdampf zugeführt oder/und erzeugt und ein Überdruck und die zur Desinfektion bzw. Sterilisation erforderliche Temperatur aufgebaut und gehalten werden können.

In der deutschen Patentanmeldung mit dem amtlichen Aktenzeichen 197 17 839.1-41 ist eine Hochtemperaturdesinfektions- bzw. Hochtemperatursterilisationsvorrichtung beschrieben, die insbesondere für krankenhausspezifische Abfälle geeignet ist und bei der die Abfälle über einen Einlaßtrichter und einen Zerkleinerer bei hintereinander angeordneten Förderschnecken zugeführt werden. Die erste Förderschnecke verläuft innerhalb einer Vorbehandlungskammer, wobei eine Aufheizung des zu behandelnden Materials in der Vorbehandlungskammer erfolgt. Diese erste Förderschnecke ist in ihrer Förderrichtung schräg nach oben geneigt angeordnet, wodurch sicher erreicht wird, daß kontaminierte Flüssigkeit, die durch den Einwurftrichter eingeführt wird, sich nur in dem Bereich unterhalb des Einwurfstrichters bzw. in einem tiefgelegenen Förderschneckenabschnitt ansammeln kann.

Die zweite Förderschnecke ist am Ende der ersten Förderschnecke angeordnet und verläuft innerhalb einer Behandlungskammer, die horizontal angeordnet ist. Innerhalb der Behandlungskammer findet der eigentliche Desinfektions- bzw. Sterilisationsprozeß statt. Dazu sind Mittel für eine Abdichtung der beiden Endbereiche der Behandlungskammer zum Aufbau eines Überdruckes vorhanden.

Bei dieser bekannten Vorrichtung sind die Vorbehandlungskammer und die Behandlungskammer einstückig miteinander verbunden. Es sind also keine Montagemittel vorhanden, mittels derer die beiden Kammern auf einfache Weise voneinander getrennt werden könnten. Dies bedeutet, daß z. B. die Kammern betreffende Reparaturarbeiten u. U. nur mit hohem Aufwand vorgenommen werden können.

Die Patentschrift US-A-3 464 342 offenbart bereits eine Vorrichtung zur Behandlung von Hühnerfedern und ähnlichem mit einer ersten, innerhalb einer Vorbehandlungskammer angeordneten Förderschnecke und einer zweiten Förderschnecke, die nahe des Endes der ersten Förderschnecke angeordnet ist und innerhalb einer Behandlungskammer verläuft. Die Vorrichtung weist Mittel zur Abdichtung der Enden der Behandlungskammer auf, wodurch in deren Inneren ein Überdruck aufgebaut werden kann. Hierzu ist mittels Dampf definiert Energre einleitbar, um den benötigten Druck und die benötigte Temperatur zum Entfernen der Feuchtigkeit aus dem eingefüllten Material bereitzustellen. Durch ein Umlenkteil, welches einerseits an dem Endabschnitt der Vorbehandlungskammer, andererseits an einem Rohrstutzen, der in die Behandlungskammer senkrecht einmündet, mittels einer Flanschverbindung lösbar fixiert ist, sind die Vorbehandlungskammer und die Behandlungskammer winklig miteinander verbunden.

Der Erfindung liegt daher die Aufgabe zugrunde, zur Behandlung von kontaminierten, insbesondere infizierten Materialien eine gattungsgemäße Vorrichtung vorzusehen, die in bezug auf ihre Montagefähigkeit und in bezug auf die Veränderbarkeit der Winkelstellung der Vorbehandlungskammer und der Behandlungskammer zueinander hohe Flexibilität bietet.

Die Aufgabe wird durch eine gattungsgemäße Vorrichtung gelöst, bei der sich die zweite Förderschnecke im wesentlichen innerhalb des gesamten Bereiches des zugehörigen Rohrabschnitts erstreckt.

Auf diese Weise ist erreicht, daß die Vorbehandlungskammer und die Behandlungskammer durch einfache Demontage des Umlenkteils voneinander getrennt werden können. Dies erleichtert z. B. ein Auswechseln einer der beiden Förderschnecken.

Ferner kann durch einen Austausch des Umlenkteils gegen ein Umlenkteil, bei dem die beiden Rohrabschnitte in einem anderen Winkel zueinander angeordnet sind, die Neigung der Vorbehandlungskammer auf einfache Weise verändert werden. Dies kann z. B. geschehen, um den Bereich innerhalb der Vorbehandlungskammer in dem eich Flüssigkeit ansammelt, zu optimieren. Darüber hinaus ist durch ein einfaches Einsetzen eines entsprechenden Umlenkteils die Übergangsfläche der beiden Rohrabschnitte leicht zu verändern. Ein weiterer Vorteil ist, daß bei Verschleiß des Umlenkteils nicht auch das Vorbehandlungsrohr ersetzt werden muß und umgekehrt.

Vorzugsweise sind die beiden Rohrabschnitte des Umlenkteils in Längsrichtung gerade. In diesem Fall kann sich beispielsweise die zweite Förderschnecke, die in der Behandlungskammer angeordnet ist, auch in den entsprechenden Rohrabschnitt des Umlenkteils erstrecken. Dieser Rohrabschnitt kann einen weiteren Flansch aufweisen, der sich an dem dem ersten Flansch gegenüberliegenden Ende des Rohrabschnittes befindet. An diesem weiteren Flansch kann z. B. ein Antrieb der zweiten Förderschnecke befestigt sein.

Das Umlenkteil kann einen Eingabestutzen für Proben aufweisen. Femer können an dem Umlenkteil auch Anschlußstutzen für Meßsensoren vorgesehen sein, um z. B. die Temperatur, den Druck, die Feuchte oder den Füllstand in der Behandlungskammer zu messen.

Ein bevorzugter Winkelbereich des Umlenkteils liegt zwischen 20° und 45°. Dieser Winkel entspricht in etwa dem Winkel, mit dem die Vorbehandiungskammer in Förderrichtung nach oben geneigt ist, da die Behandlungskammer vorzugsweise horizontal angeordnet ist.

Innerhalb des Umlenkteils sind vorzugsweise Mittel zum Abdichten der Behandlungskammer angeordnet. Durch die einfache Austauschbarkeit des Umlenkteils können somit auch die Abdichtungsmittel auf einfache Weise gewechselt werden. Dies ermöglicht eine hohe Flexibilität bei der Verwendung der Vorrichtung. So kann beispielsweise im Endbereich der ersten Förderschnecke innerhalb des Umlenkteils ein konusförmiger Ring derartig angeordnet sein, daß eine Verdichtung des Materials stattfindet.

Die erste Förderschnecke ist vorzugsweise von einem Heizelement umgeben, um das zu behandelnde Material während der Beförderung durch die Schnecke aulzuheizen.

Im folgenden wird die Erfindung anhand eines Ausführungsbeispiels der erfindungsgemäßen Vorrichtung näher erläutert, wobei auf die Figuren Bezug genommen wird. Es zeigen:
Figur 1, eine schematische Seitenansicht eines erfindungsgemäßen Umlenkteils, das mit einer Vorbehandlungskammer und einer Behandlungskammer verbunden ist, die jedoch nur im Ansatz gezeigt sind;
Figur 2, eine schematisch Draufsicht auf das Umlenkteil gemäß Figur 1;
Figur 3, eine schematische Gesamtdarstellung der das Umlenkteil gemäß Figur 1 aufweisenden erfindungsgemäßen Vorrichtung.

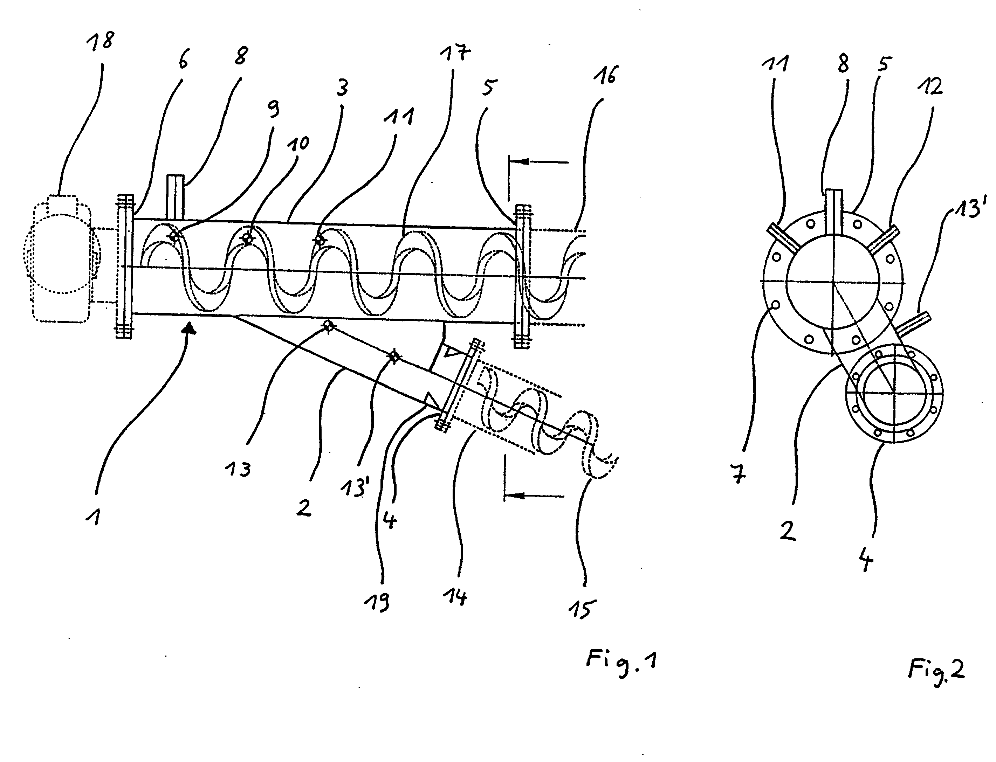

In Figur 1 ist ein Umlenkteil 1 dargestellt, das zwei Rohrabschnitte 2 und 3 aufweist. Die beiden Rohrabschnitte 2 und 3 sind kommunizierend miteinander verbunden.

Der Rohrabschnitt 2 weist an seinem freien Ende einen Flansch 4 auf. Der Rohrabschnitt 3 besitzt an seinen beiden Enden ebenfalls jeweils einen Flansch 5 bzw. 6. Diese Flansche 4, 5 und 6 besitzen Öffnungen, z. B. bezeichnet mit 7 (Fig. 2), um verschraubt werden zu können.

Der Rohrabschnitt 3 ist mit einem Stutzen 8 zur Eingabe von Proben versehen. Ferner weist der Rohrabschnitt 3 in Längsrichtung auf jeder Seite drei Stutzen zum Anschließen von Meßsensoren auf, von denen die Stutzen 9, 10, 11 und 12 in der Figur 1 bzw. Figur 2 gezeigt sind. Zwei weitere Stutzen 13 und 13' zum Anschließen von Meßsensoren weist der Rohrabschnitt 2 auf.

Das Umlenkteil 1 weist ferner einen konusförmigen Ring 19 auf, der den freien Durchlaßquerschnitt des Rohrabschnitts 2 in Förderrichtung verringert.

In Figur 1 sind weitere Komponenten einer erfindungsgemäßen Vorrichtung gestrichelt gezeichnet. Es handelt sich hierbei um eine nur teilweise gezeigte Vorbehandlungskammer 14, an der der Rohrabschnitt 2 über den Flansch 4 befestigt ist. In der Vorbehandlungskammer 14 befindet sich eine erste Förderschnecke 15, die sich auch bis in das Übergangsstück hineinerstrecken kann.

Der Rohrabschnitt 3 ist mit dem Flansch 5 an einer Behandlungskammer 16 befestigt. Innerhalb der Behandlungskammer 16 und dem Rohrabschnitt 3 verläuft eine zweite Förderschnecke 17. Die zweite Förderschnecke 17 wird durch einen Antrieb 18 bewegt, der an dem Rohrabschnitt 3 mittels des Flansches 6 befestigt ist.

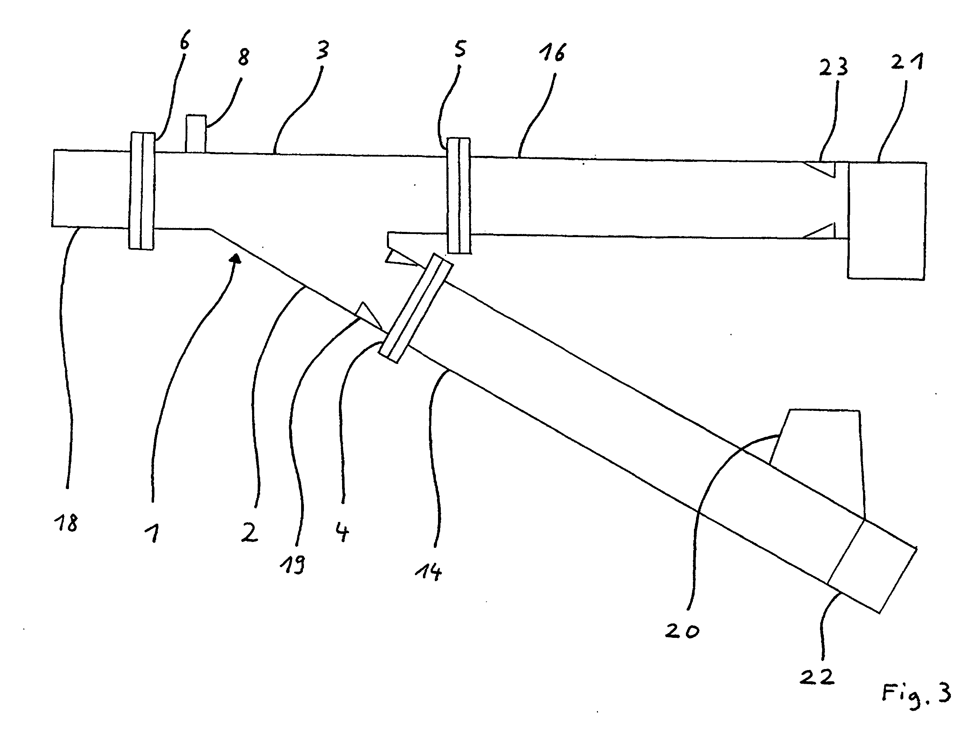

In Figur 3 ist die Vorrichtung schematisch in Gesamtdarstellung gezeigt. Insbesondere sind in dieser Darstellung eine Eingabeeinheit 20 mit einem nicht gezeigten Zerkleinerer und ein Auswurf 21 und ein Antrieb 22 der ersten Förderschnecke 15 zu sehen. Femer ist ein weiterer konusförmiger Ring 23 gezeigt, der ausgangsseitig innerhalb der Behandlungskammer 16 angeordnet ist.

Der in den Figuren dargestellten Ausführungsform der Erfindung liegt folgende Funktionsweise zugrunde.

Kontaminiertes Material wird über die Eingabeeinheit 20 der ersten Förderschnecke 15, die sich innerhalb der Vorbehandlungskammer 14 erstreckt, zugeführt. Die Förderschnecke 15 ist von einem Heizelement (nicht gezeigt) umgeben, so daß während der Beförderung durch die Förderschnecke 15 das zu behandelnde Material definiert aufgeheizt wird. In der Förderschnecke 15 erfolgt ferner eine Verdichtung des Materials in der Form, daß in deren Endbereich ein abdichtender Materialpfropfen erzeugt wird. Dies geschieht zum einen dadurch, daß der Steigungswinkel der Förderschnecke 15 in deren Endbereich verringert ist. Zum anderen wird die Verdichtung durch den konusförmigen Ring 19 erzielt.

Die Förderschnecke 15 transportiert das Material zu der zweiten Förderschnecke 17, die sich im wesentlichen innerhalb der Behandlungskammer 16, aber auch innerhalb des Rohrabschnitts 3 erstreckt. Innerhalb der Behandlungskammer 16 erfolgt eine Entspannung und eine Auflockerung der Struktur des zu behandelnden Materials. Die zweite Förderschnecke 17 ist ebenfalls von einem Heizelement umgeben und ist zur eigentlichen Behandlung des Materials ausgebildet. In die Behandlungskammer 16 kann Energie stoßweise eingeleitet werden, femer kann Wasserdampf zugeführt oder/und erzeugt und ein Überdruck und die für die Desinfektion bzw. Sterilisation erforderliche Temperatur aufgebaut und gehalten werden. Die Förderschnecke 17 verdichtet das Material in ihrem Endbereich wiederum aufgrund einer verringerten Steigung der Schnecke 17 und mittels des konusförmigen Ringes 23 zu einem zweiten abdichtenden Materialpfropfen. Dadurch kann zwischen den beiden als Dichtung wirkenden Materialpfropfen der Förderschnecken 15, 17 der für eine Desinfektion bzw. Sterilisation erforderliche Überdruck für einen definierten Zeitraum gehalten werden.

Da die erste Förderschnecke 15 in einem Winkel von ca. 30° zur horizontalen Ebene angeordnet ist, ergibt sich eine Aufwärtsförderung des Materials. Hierdurch wird sehr sicher verhindert, daß kontaminierte Flüssigkeit aus dem Bereich unterhalb der Eingabeeinheit unbemerkt in die Anlage fließt und den Prozeß unbehandelt bzw. unzulänglich behandelt passieren kann.

Dadurch, daß das Umlenkteil 1 einfach austauschbar ist, können verschiedene Parameter der erfindungsgemäßen Vorrichtung auf einfache Weise verändert werden. Hierzu gehören z. B. die Steigung der ersten Förderschnecke 15 und die Mittel 19 zur Abdichtung der Behandlungskammer 16 in dem Umlenkteil 1. Mit der erfindungsgemäßen Vorrichtung können kontaminierte Materalien, bei denen es sich vorzugsweise um krankenhausspezifische Abfälle, aber z. B. auch um Klärschlamm, kontaminierte Böden sowie Lebensmittel, wie Getreide und Gewürze, handeln kann, sicher desinfiziert und je nach Anforderung und je nach Auslegung der Anlage auch sterilisiert werden.

## Patentansprüche

1. Vorrichtung zur Desinfektion bzw. Sterilisation von kontaminierten, insbesondere infizierten Materialien, mit einer ersten Förderschnecke (15), die innerhalb einer Vorbehandlungskammer (14) verläuft und einer zweiten Förderschnecke (17), die nahe des Endes der ersten Förderschnecke (15) angeordnet ist und innerhalb einer Behandlungskammer (16) verläuft, wobei Mittel (19, 23) für eine Abdichtung der beiden Endbereiche der Behandlungskammer (16) zum Aufbau eines Überdruckes vorhanden sind, in die Behandlungskammer (16) Energie defintert einleitbar ist, Wasserdampf zugeführt oder/und erzeugt und ein Überdruck und die zur Desinfektion bzw. Sterilisation erforderliche Temperatur aufgebaut und gehalten werden können, und wobei die Vorbehandlungskammer (14) und die Behandlungskammer (16) durch ein Umlenkteil (1) miteinander verbunden sind, das zwei in einem Winkel zueinander angeordnete Rohrabschnitte (2, 3) aufweist und an der Vorbehandlungskammer (14) und der Behandlungskammer (16) durch Flansche (4, 5) lösbar befestigt ist, **dadurch gekennzeichnet, daß** sich die zweite Förderschnecke (17) im wesentlichen innerhalb des gesamten Bereiches des zugehörigen Rohrabschnitts (3) erstreckt.

2. Vorrichtung nach Anspruch 1, **dadurch gekennzeichnet, daß** die Rohrabschnitte (2, 3) geradlinig verlaufen.

3. Vorrichtung nach einem der vorhergehenden Ansprüche, **dadurch gekennzeichnet, daß** der mit der Behandlungskammer (16) unmittelbar verbundene Rohrabschnitt (3) einen weiteren, dem ersten gegenüberliegenden Flansch (6) aufweist.

4. Vorrichtung nach Anspruch 3, **dadurch gekennzeichnet, daß** an dem weiteren Flansch (6) ein Antrieb (18) der zweiten Förderschnecke (17) befestigt ist.

5. Vorrichtung nach einem der vorhergehenden Ansprüche, **dadurch gekennzeichnet, daß** das Umlenkteil (1) einen Eingabestutzen (8) für Proben aufweist.

6. Vorrichtung nach einem der vorhergehenden Ansprüche, **dadurch gekennzeichnet, daß** das Umlenkteil (1) mindestens einen Anschlußstutzen (9, 10, 11, 12, 13, 13') für Meßsensoren zur Messung der Temperatur, des Druckes, der Feuchte und/oder des Füllstandes aufweist.

7. Vorrichtung nach einem der Ansprüche 2 bis 6, **dadurch gekennzeichnet, daß** der Winkel des Umlenkteils (1) 20° bis 45° beträgt, wobei die Vorbehandlungskammer (14) in Förderrichtung schräg nach oben geneigt angeordnet ist.

8. Vorrichtung nach einem der vorhergehenden Ansprüche, **dadurch gekennzeichnet, daß** Mittel (19) zum Abdichten der Behandlungskammer innerhalb des Umlenkteils (1) angeordnet sind.

9. Vorrichtung nach Anspruch 8, **dadurch gekennzeichnet, daß** im Endbereich der ersten Förderschnecke (15) innerhalb des Umlenkteils (1) ein konusförmiger Ring (19) derartig angeordnet ist, daß eine Verdichtung des zu behandelnden Materials erfolgt.

## Claims

1. Device for disinfecting and sterilising contaminated, in particular infected, materials comprising a first conveyor screw (15) extending inside a pre-treatment chamber and a second conveyor screw (17) arranged in the vicinity of the end of the first conveyor screw (15) and extending inside a treatment chamber (16), wherein there are means (19, 23) for sealing the two end regions of the treatment chamber (16) to create an excess pressure, energy can be introduced into the treatment chamber (16) in a defined manner, water vapour can be supplied and/or generated and an excess pressure and the temperature required for disinfection and sterilisation built up and maintained, the pre-treatment chamber (14) and the treatment chamber (16) being connected to one another via a deflecting part (1) with two tubular portions (2, 3) arranged at an angle to one another and detachably fastened to the pre-treatment chamber (14) and the treatment chamber (16) by flanges (4, 5), **characterised in that** the second conveyor screw (17) extends substantially inside the entire region of the associated tubular portion (3).

2. Device according to claim 1, **characterised in that** the tubular portions (2, 3) extend linearly.

3. Device according to any of the preceding claims, **characterised in that** the tubular portion (3) directly connected to the treatment chamber (16) has a further flange (6) opposing the first flange.

4. Device according to claim 3, **characterised in that** a drive (18) of the second conveyor screw (17) is fastened to the further flange (6).

5. Device according to any of the preceding claims, **characterised in that** the deflecting part (I) has an input connection piece (8) for samples.

6. Device according to any of the preceding claims, **characterised in that** the deflecting part (1) has at least one connecting sleeve (9, 10, 11, 12, 13, 13') for measuring sensors to measure the temperature, the pressure, the humidity and/or the level.

7. Device according to any of claims 2 to 6, **characterised in that** the angle of the deflecting part (1) is 20° to 45°, the pre-treatment chamber (14) being arranged so as to be obliquely upwardly inclined in the conveying direction.

8. Device according to any of the preceding claims, **characterised in that** means (19) for sealing the treatment chamber are arranged inside the deflecting part (1).

9. Device according to claim 8, **characterised in that** a conical ring (19) is arranged in the end region of the first conveyor screw (15), inside the deflecting part (1), in such a way that the material to be treated is compacted.

## Revendications

1. Dispositif pour la désinfection ou la stérilisation de matériaux contaminés, en particulier infectés, grâce à une première vis sans fin (15), qui passe à l'intérieur d'une chambre de traitement initial (14) et d'une deuxième vis sans fin (17), qui est disposée à proximité de l'extrémité de la première vis sans fin (15) et passe à l'intérieur d'une chambre d'irradiation (16), tandis que l'on dispose de moyens (19, 23) pour une étanchéification des deux secteurs terminaux de la chambre d'irradiation (16) dans le but de développer une surpression, et tandis qu'une énergie définie peut être introduite dans la chambre d'irradiation (16), que de la vapeur d'eau peut être amenée et/ou produite et qu'une surpression et la température nécessaire à la désinfection ou à la stérilisation peuvent être établies ou conservées, et tandis que la chambre de traitement initial (14) et la chambre d'irradiation (16) sont reliées entre elles par une chicane déflectrice (1) qui présente deux segments de tube (2, 3) agencés l'un par rapport à l'autre selon un angle, et qui sont fixés à la chambre de traitement initial (14) et à la chambre d'irradiation (16) par des brides (4, 5) démontables, **caractérisé en ce que** la deuxième vis sans fin (17) s'avance essentiellement à l'intérieur de l'ensemble du secteur des segments de tube (3) apparentés.

2. Dispositif selon la revendication 1, **caractérisé en ce que** les segments de tube (2, 3) passent en ligne droite.

3. Dispositif selon l'une des revendications précédentes, **caractérisé en ce que** le segment de tube (3) directement relié à chambre d'irradiation (16) présente une autre bride située à l'opposé.

4. Dispositif selon la revendication 3, **caractérisé en ce que** sur l'autre bride (6) est fixé un entraînement (18) de la deuxième vis sans fin (17).

5. Dispositif l'une des revendications précédentes, **caractérisé en ce que** la chicane déflectrice (1) présente un tube de remplissage (8) pour prélèvements.

6. Dispositif selon l'une des revendications précédentes, **caractérisé en ce que** la chicane déflectrice (1) présente au moins un raccord (9, 10, 11, 12, 13, 13') pour capteurs de mesure pour la mesure de la température, de la pression, de l'humidité et/ou de l'état du remplissage.

7. Dispositif selon l'une des revendications 2 à 8, **caractérisé en ce que** l'angle de la chicane déflectrice (1) a une valeur située entre 20° et 45°, tandis que la chambre de traitement initial (14) est agencée en diagonale, inclinée vers le haut en direction du déplacement.

8. Dispositif selon l'une des revendications précédentes, **caractérisé en ce que** des moyens (19) pour l'étanchéification de la chambre de traitement sont agencés à l'intérieur de la chicane déflectrice (1).

9. Dispositif selon la revendication 8, **caractérisé en ce que** dans le secteur terminal de la première vis sans fin (15) à l'intérieur de la chicane déflectrice (1) un anneau de forme conique (19) est agencé de telle manière qu'il se produit une compression du matériau à traiter.
